Europäisches Patentamt

European Patent Office

Office européen des brevets

(11) Publication number: **0 059 422**
**B1**

(12) EUROPEAN PATENT SPECIFICATION

(45) Date of publication of patent specification: **17.07.85**

(51) Int. Cl.⁴: **B 01 J 23/66, C 07 D 301/10**

(21) Application number: **82101391.9**

(22) Date of filing: **24.02.82**

(54) **Silver-based catalyst for production of ethylene oxide.**

(30) Priority: **25.02.81 JP 25417/81**
**30.04.81 JP 65435/81**

(43) Date of publication of application:
**08.09.82 Bulletin 82/36**

(45) Publication of the grant of the patent:
**17.07.85 Bulletin 85/29**

(84) Designated Contracting States:
**DE GB NL**

(56) References cited:
**DE-A-2 454 972**
**FR-A-2 351 700**
**GB-A-2 043 481**
**US-A-2 765 283**
**US-A-2 799 687**

(73) Proprietor: **MITSUBISHI PETROCHEMICAL CO., LTD.**
**5-2, 2-chome, Marunouchi**
**Chiyoda-ku Tokyo 100 (JP)**

(72) Inventor: **Nojiri, Naohiro**
**c/o Chuo Kenkyujo 1315, Oaza Wakaguri**
**Ami-machi Inashiki-gun Ibaraki-ken (JP)**
Inventor: **Sakai, Yukio**
**c/o Chuo Kenkyujo 1315, Oaza Wakaguri**
**Ami-machi Inashiki-gun Ibaraki-ken (JP)**
Inventor: **Ayame, Akimi**
**32-2-103, Mizumoto-cho**
**Muroran-shi Hokkaido (JP)**

(74) Representative: **Kraus, Walter, Dr. et al**
**Patentanwälte Kraus Weisert & Partner Thomas-**
**Wimmer-Ring 15**
**D-8000 München 22 (DE)**

Courier Press, Leamington Spa, England.

**Description**

This invention relates to a catalyst for the production of ethylene oxide by oxidation of ethylene comprising (A) silver, (B) (1) sodium and (2) cesium as a cationic component, and (C) at least one halogen element selected from the group consisting of chlorine, bromine and fluorine as an anionic component. This invention relates also to a process for the production of ethylene oxide using said catalyst. The invention is based on the new discovery that a combination of sodium, cesium, and a specified halogen element (chlorine, bromine and/or fluorine) with silver provides a catalyst having increased performance in the oxidation of ethylene.

A silver catalyst is substantially the sole catalyst component used in the industrial production of ethylene oxide by oxidation of ethylene with molecular oxygen. Silver alone, however, did not prove to be a perfect industrial catalyst, and efforts have been made to increase its performance by including various additives (for example, US—A—2 238 471, 2 404 438, 2 671 764 and 2 799 687). Many of these efforts have been directed to the addition of alkali metals (for example, GB—A—1 413 251 and US—A—4 212 772).

GB—A—2 043 481 discloses a catalyst for the production of ethylene oxide by oxidation of ethylene, which comprises silver, sodium and cesium as cationic component and one halogen element as an anionic component.

The present inventors have extensively made investigations about the effects of anionic components as well as alkali metals as cationic components. These investigations have led to the discovery that the use of the aforesaid specified halogen elements (to be referred to simply as a halogen element), previously believed to have a poisonous action, in combination with sodium and cesium unexpectedly gives a catalyst having greatly increased performance, particularly a higher selectivity at lower reaction temperatures.

According to this invention, there is provided a catalyst as mentioned above which is characterized by the fact that

(1) said sodium is contained in an amount of more than 1000 ppm (mg/kg of catalyst) based on the catalyst,

(2) said cesium is contained in an amount of from 10 ppm to 0.5% by weight based on the catalyst, and

(3) said halogen element is contained in an amount of from 5 ppm to 0.1% by weight based on the catalyst.

In another aspect, there is provided a process for the production of ethylene oxide, which comprises contacting ethylene and a gas containing molecular oxygen at a temperature of 180 to 300°C and a pressure of 1 to 35 kg/cm$^2$ with a catalyst comprising

(A) silver,

(B) (1) sodium and (2) cesium as a cationic component, and

(C) at least one halogen element selected from the group consisting of chlorine, bromine and fluorine as an anionic component which process is characterized by the fact that a catalyst is used in which

(1) said sodium is contained in an amount of more than 1000 ppm (mg/kg of catalyst) based on the catalyst,

(2) said cesium is contained in an amount of from 10 ppm to 0.5% by weight based on the catalyst, and

(3) said halogen element is contained in an amount of from 5 ppm to 0.1% by weight based on the catalyst.

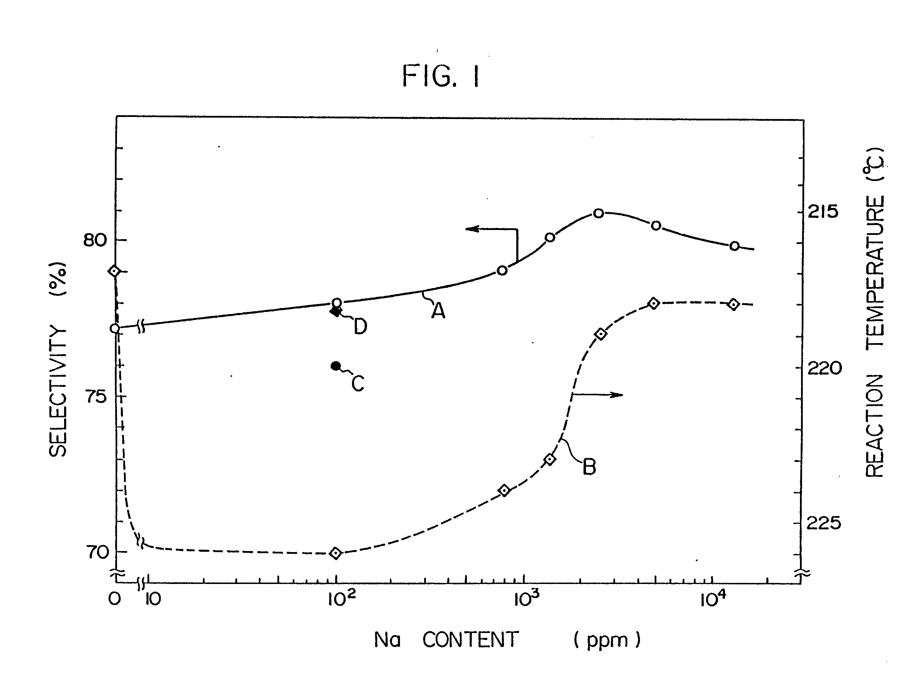

Figure 1 is a graph showing the effect of the sodium contents of the catalysts used in Examples 7 and 10 and Comparative Examples 6 to 9 on the selectivities of the catalysts and the reaction temperatures.

In the preparation of the catalyst of this invention, a sodium compound to be added is preferably in the form of sodium carbonate, sodium bicarbonate or sodium nitrate. It may, however, be in the form of a hydroxide, nitrite, sulfate, borate, silicate, phosphate, halide or carboxylate (e.g., sodium acetate or sodium oxalate), or a mixture of any of these compounds with sodium carbonate or sodium bicarbonate.

A cesium compound to be added is preferably in the form of a chloride, bromide and/or fluoride depending upon the type of the halogen element to be added to the catalyst. It may be added in the form of any of water-soluble or water-insoluble compounds such as its hydroxide or salts such as nitrate, nitrite, carbonate and sulfate.

Sources of the halogen element may be sodium halides or cesium halides which are salts of halogens with sodium or cesium, or other salts such as $BaCl_2$, $NH_4Cl$, LiCl, KCl, RbCl, $BaBr_2$, $BaF_2$, KBr, KF, RbBr, RbF, LiBr and LiF.

In any case, it is essential to add sodium, cesium and the halogen element as well as silver, and a combination of these gives a catalyst having greatly increased performance.

The amount of solution added is more than 1000 ppm (mg/kg of catalyst) based on the catalyst. Preferably, it is from 1100 ppm to 1.50% by weight, more preferably 1200 ppm to 1% by weight. Advantageously, the amount of sodium added ranges from 1300 ppm to 5000 ppm. Preferably, the amount of sodium is such that the atomic ratio of Na to Ag is not more than 0.58, especially not more than 0.45. When the amount of sodium is too large, both the activity and selectivity of the catalyst are reduced. When it is too small, the dispersion of silver particles on a carrier is worse than that in the catalyst of this invention as observed by a scanning electron microscope; hence, the activity of the catalyst is low and a higher bath temperature is required in order to obtain the same activity, with the consequence that the effect of adding halogen is not fully produced.

2

The suitable amount of cesium added is smaller than that of sodium. It is from 10 ppm to 0.5% by weight, preferably from 15 ppm to 0.1% by weight, based on the catalyst. If the amount is too large, the activity of the catalyst is markedly decreased, and if it is too small, the effect of the halogen element is not fully produced.

The suitable amount of the specified halogen element, i.e. chlorine, bromine and/or fluorine, used in the catalyst of this invention is from 5 ppm to 0.1% by weight, preferably from 7 ppm to 0.07% by weight. The addition of too large an amount results in the exhibition of its poisoning action and causes a drastic reduction in the performance of the catalyst. Thus, the characteristic feature of the invention is that the halogen element previously believed to have a poisoning action has been found to have an action of a performance improver by adding it in a very small amount in combination with sodium and cesium.

The halogen elements may be used singly or as a mixture of two or three of them.

In the catalyst of this invention, the cationic component is not limited to sodium and cesium, and a small amount of a third component such as lithium, barium rubidium, potassium and thallium may be added together.

The above catalyst components are used in the form of a supported catalyst mainly from the standpoint of economy and active lifetime. A porous refractory material is used as a carrier. Desirably, the carrier has a BET surface area of 0.05 to 10 $m^2/g$ and an apparent porosity of at least 15%. A carrier composed of alpha-alumina as a main component (a so-called alundum carrier) is preferred.

Deposition of sodium, cesium and the halogen element may be effected by dissolving or dispersing the aforesaid compounds of these ingredients in an aqueous solvent, impregnating a carrier with the resulting solution or dispersion, and drying the impregnated carrier under heat in the presence of a gas such as nitrogen or air. Sodium, cesium and the halogen element may be deposited simultaneously or separately in any desired stage of catalyst preparation in various modes. For example, similar effects can be obtained by performing the deposition before, during or after the impregnation of the silver compound.

Deposition of silver can be carried out by dipping a carrier molded in a suitable form such as spheres, pellets or rings in an aqueous solution or dispersion prepared by dissolving or dispersing a silver compound such as silver oxalate, silver nitrate or silver lactate in water in the presence or absence of a solubilizing agent such as ethylenediamine, drying the impregnated carrier, and calcining it at a suitable temperature in a stream of a gas such as nitrogen, air or hydrogen. The kind of the gas and the temperature in the calcination are selected depending upon the kind of the silver salt, etc. The calcination temperature is usually 100 to 1000°C, preferably 150 to 700°C. The amount of silver supported in the catalyst is usually 1 to 25% by weight, preferably 3 to 20% by weight, based on the catalyst.

The silver-based catalyst of this invention is used conveniently in the production of ethylene oxide by the oxidation of ethylene in the vapor phase with molecular oxygen. The reaction conditions for the oxidation of ethylene are known, and broadly described in the prior art literature. In the production of ethylene oxide in the presence of the silver-based catalyst of this invention, the reaction pressure is 1 to 35 $kg/cm^2$, preferably 5 to 20 $kg/cm^2$; the reaction temperature is 180 to 300°C, preferably 190 to 260°C; ethylene is used in an amount of 1 to 40% by volume, preferably 15 to 35% by volume; and oxygen is used in an amount of 1 to 20% by volume, preferably 5 to 10% by volume, diluted with a diluting agent such as methane and nitrogen, preferably methane, in an amount of 20 to 70% by volume, preferably 30 to 65% by volume. Oxygen may be supplied in the form of air or industrial oxygen. Preferably, a reaction inhibitor such as a halogenated hydrocarbon is added to the starting gaseous mixture. In particular, by adding several ppm to several ten ppm (by weight) of ethylene dichloride, vinyl chloride, etc. to the starting gas, the formation of hot spots in the catalyst can be prevented and the properties, especially the selectivity, of the catalyst can be greatly improved. The starting gaseous mixture is continuously introduced into a reactor filled with the catalyst. The resulting ethylene oxide is separated and recovered from the reaction mixture by using customary methods.

The following examples illustrate the present invention more specifically.

Example 1

$AgNO_3$ (6.0 g) was dissolved in 100 ml of water, and separately, 3.4 g of potassium oxalate ($K_2C_2O_4 \cdot H_2O$) was dissolved in water. The resulting solutions were mixed, and heated in a water bath to 60°C to form a white precipitate of silver oxalate. Centrifugation and washing with distilled water were repeated to remove potassium from the precipitate.

Separately, 6.6 ml of 1:1 mixture of ethylenediamine and water was prepared, and while cooling with ice, the silver oxalate precipitate was gradually dissolved in the mixture to prepare a silver solution. Thirty grams of an alpha-alumina carrier (SA-5161, a tradename for a product of Norton Company) was dipped in the silver solution, and after allowing the excess of the solution to flow out, was dried at 80°C under reduced pressure in a rotary evaporator. The dried carrier was transferred into a calcination tube. The temperature was raised to 300°C in a stream of nitrogen over the course of 2 hours, and it was further calcined at this temperature for 2 hours to deposit silver on the carrier.

Then, 2.0 g of $Na_2CO_3$ and 0.04 g of CsCl were dissolved in 50 ml of a mixture of methanol and water (methanol content 30% by weight), and the silver-supported carrier was dipped in it. The excess of the solution was removed by filtration, and the impregnated carrier was dried at 110°C for 2 hours in a stream of nitrogen gas to prepare a catalyst in accordance with this invention. The proportion of Ag supported was 8.0% by weight, and the proportions of Na, Cs and Cl supported were 0.35% by weight, 126 ppm, and 34 ppm, respectively.

The catalyst was pulverized to a size of 9 to 28 mesh, and 10 g of the pulverized catalyst was filled in a steel reactor having an inside diameter of 20 mm. A gaseous mixture consisting of 30% by volume of ethylene, 8% by volume of oxygen, 2 ppm of vinyl chloride and the balance being methane was passed through the reactor at a pressure of 9 kg/cm² and an SV of 2000 hr⁻¹. At a bath temperature (reaction temperature) of 200°C, an oxygen conversion of 35% and an ethylene oxide selectivity of 81.0% were obtained. At 207°C, the oxygen conversion was 61.0% and the ethylene oxide selectivity was 75%. During the three-week continuous operation, no change was noted in the performance of the catalyst.

## Example 2

Thirty grams of an alpha-alumina carrier (SA-5161) was dipped in 50 ml of an aqueous solution containing 2.0 g of sodium carbonate. The excess of the solution was removed by filtration, and the impregnated carrier was dried at 110°C for 2 hours in a stream of nitrogen gas to prepare a carrier impregnated with sodium carbonate.

Silver was deposited on the carrier in the same way as in Example 1. The carrier was then dipped in a solution of 0.02 g of cesium chloride in 50 ml of a mixture of methanol and water (water content 0.3% by weight). The excess of the solution was removed, and the impregnated carrier was dried at 110°C for 2 hours in a stream of nitrogen gas to prepare a catalyst in accordance with this invention. The proportion of Ag deposited was 8.0% by weight, and the proportions of Na, Cs and Cl deposited were 0.35% by weight, 63 ppm and 17 ppm, respectively.

Ethylene was oxidized under the same conditions as in Example 1 using the resulting catalyst. At a reaction temperature of 195°C, an oxygen conversion of 32% and an ethylene oxide selectivity of 80.0% were obtained.

## Comparative Example 1

The procedure of Example 1 was followed except that the amount of sodium carbonate was decreased to one-tenth, and the amount of cesium chloride added was decreased to one-half. Thus, there was prepared a supported catalyst containing 8.0% by weight of Ag, 350 ppm of Na, 63 ppm of Cs and 17 ppm of Cl. Ethylene was oxidized under the same conditions as in Example 1 using the resulting catalyst. At a reaction temperature of 195°C, an oxygen conversion of 23% and an ethylene oxide selectivity of 79.5% were obtained.

## Example 3

Silver was deposited on a carrier (SA-5161) in the same was as in Example 1.

The silver-supported carrier was then dipped in a solution of 2.0 g of sodium carbonate, 0.13 g of cesium nitrate and 0.014 g of sodium chloride in 50 ml of a mixture of methanol and water (methanol content 30% by weight). The excess of the solution was removed, and the impregnated carrier was dried at 110°C for 2 hours in a stream of nitrogen gas to prepare a catalyst in accordance with this invention.

The proportion of Ag deposited was 8% by weight, and the proportions of Na, Cs and Cl deposited were 0.35% by weight, 315 ppm, and 34 ppm, respectively.

Ethylene was oxidized under the same conditions as in Example 1 using the resulting catalyst. At a reaction temperature of 210°C, an oxygen conversion of 34.5% and an ethylene oxide selectivity of 80.0% were obtained.

## Example 4

A supported catalyst containing 8.0% by weight of Ag, 0.22% by weight of Na, 126 ppm of Cs and 34 ppm of Cl was prepared in the same way as in Example 1 except that 2.0 g of sodium bicarbonate was used instead of sodium carbonate. Ethylene was oxidized under the same conditions as in Example 1 using the resulting catalyst. At a reaction temperature of 215°C, an oxygen conversion of 30.0% and an ethylene oxide selectivity of 81.0% were obtained.

## Example 5

A supported catalyst containing 8.0% by weight of Ag, 0.23% by weight of Na, 126 ppm of Cs and 34 ppm of Cl was prepared in the same way as in Example 1 except that 2.0 g of sodium acetate was used instead of sodium carbonate. Ethylene was oxidized under the same conditions as in Example 1 using the resulting catalyst. At a reaction temperature of 218°C, an oxygen conversion of 30.0% and an ethylene oxide selectivity of 81.0% were obtained.

## Example 6

A supported catalyst containing 8.0% by weight of Ag, 0.24% by weight of Na, 126 ppm of Cs and 34 ppm of Cl was prepared in the same way as in Example 1 except that 2.0 g of sodium nitrate was used instead of sodium carbonate. Ethylene was oxidized under the same reaction conditions as in Example 1 using the resulting catalyst. At a reaction temperature of 219°C, and oxygen conversion of 34.0% and an ethylene oxide selectivity of 80.5% were obtained.

### Comparative Examples 2 to 5

Four different catalysts having the compositions shown in Table 1 were prepared in the same way as in Example 1, and tested under the same reaction conditions as in Example 1. The results together with those obtained in Example 1 are summarized in Table 1. It is seen from Table 1 that the catalyst of this invention shows higher selectivity.

TABLE 1

| Run | | Composition of the catalyst | Raw materials added | Reaction temperature (°C) | Oxygen conversion (%) | Selectivity (%) |
|---|---|---|---|---|---|---|
| Example 1 | | Ag——Na——Cs——Cl<br>8   0.35  126  34<br>wt%  wt%  ppm  ppm | $Na_2CO_3$,<br>CsCl | 207 | 61.0 | 75 |
| Comparative Example | 2 | Ag——Na<br>8   0.35<br>wt%  wt% | $Na_2CO_3$ | 205 | 60.2 | 70.3 |
| | 3 | Ag——Cs——Cl<br>8  126  34<br>wt%  ppm  ppm | CsCl | 228 | 60.0 | 68.0 |
| | 4 | Ag——Na——Cs<br>8  0.35  126<br>wt%  wt%  ppm | $Na_2CO_3$,<br>$CsNO_3$ | 200 | 58.5 | 67.7 |
| | 5 | Ag——Na——Cl<br>8  0.35  34<br>wt%  wt%  ppm | $Na_2CO_3$,<br>NaCl | 206 | 68.0 | 66.5 |

### Example 7

Thirty grams of an alpha-alumina carrier (SA-5161) was dipped in 50 ml of an aqueous solution containing 2.2 g of sodium bicarbonate. The excess of the solution was removed by filtration, and the impregnated carrier was dried at 110°C for 2 hours in a stream of nitrogen gas to prepare a sodium bicarbonate-impregnated carrier.

Silver nitrate (6.0 g) was dissolved in 100 ml of water, and 3.4 g of potassium oxalate ($K_2C_2O_4 \cdot H_2O$) was dissolved in 100 ml of water. The aqueous solutions were mixed, and heated to 60°C in a water bath to form a white precipitate of silver oxalate. Centrifugation and washing with distilled water was repeated to remove potassium from the precipitate.

Separately, 6.6 ml of a 1:1 mixture of ethylenediamine and water was prepared, and while cooling with ice, the precipitate of silver oxalate was gradually dissolved in the mixture to prepare a silver solution. The above carrier impregnated with sodium bicarbonate was dipped in the resulting silver solution. The excess of the solution was allowed to flow out, and the carrier was dried at 80°C under reduced pressure in a rotary evaporator. The dried carrier was transferred to a calcination tube and the temperature was raised to 300°C over the course of 2 hours. It was further calcined at this temperature for 1.5 hours. The calcine product was cooled, and dipped in 50 ml of a methanol/water mixture (water content 0.3% by weight) containing 0.043 g of cesium chloride. The excess of the solution was removed by filtration, and the impregnated carrier was dried at 110°C for 2 hours in a stream of nitrogen to prepare a catalyst in accordance with this invention which had the composition shown in Table 2.

The catalyst was tested under the same reaction conditions as in Example 1. The results are shown in Table 2.

### Examples 8 to 10

In each run, a catalyst was prepared in the same way as in Example 7 except that the content of Na was changed as shown in Table 2. The resulting catalyst was tested in the same was as in Example 7. The results are shown in Table 2.

TABLE 2

| | | Composition of the catalyst | | | | Results of the reaction | | |
|---|---|---|---|---|---|---|---|---|
| | | Ag (wt%) | Na (ppm) | Cs (ppm) | Cl (ppm) | Temperature (°C) | Oxygen conversion (%) | Selectivity (%) |
| Example | 7 | 8.4 | 2500 | 160 | 42 | 219 | 40 | 80.8 |
| | 8 | 8.4 | 1380 | 160 | 42 | 223 | 40 | 80.2 |
| | 9 | 8.9 | 5000 | 150 | 40 | 218 | 40 | 80.5 |
| | 10 | 8.4 | 13000 | 160 | 42 | 218 | 40 | 79.8 |
| Comparative Example | 6 | 8.3 | 0 | 160 | 42 | 217 | 40 | 77.2 |
| | 7 | 8.4 | 100 | 160 | 42 | 226 | 40 | 78.1 |
| | 8 | 8.4 | 800 | 160 | 42 | 224 | 40 | 78.9 |
| | 9 | 8.4 | 100 | 50 | 13 | 218 | 40 | 76.0 |

### Comparative Examples 6 to 8

In each run, a catalyst was prepared in the same way as in Example 7 except that the content of Na was outside the scope of the invention as shown in Table 2. The resulting catalyst was tested in the same way as in Example 7. The results are shown in Table 2. It is seen from Table 2 that the catalysts having an Na content outside the scope of the invention showed inferior selectivity to the catalyst of this invention.

### Comparative Example 9

A catalyst containing Cs and Na as cationic components in the same proportions as in Experiment 5C of Japanese Laid-open Patent Publication No. 127144/1980 and chlorine as an ionic component was repeated by the same catalyst preparing method using the same raw materials as in Example 7. The catalyst was tested under the same reaction conditions as in Example 7. The results are shown in Table 2. It is seen that the catalyst was inferior in selectivity to the catalyst of this invention.

The effect of the Na contents of the catalysts shown in Table 2 on the selectivity and reaction temperature is plotted on a graph shown in Figure 1, in which the axis of abscissas represents the Na content (ppm) on a logarithmic scale and axes of ordinates, the left and right ones, represent the selectivity (%) for ethylene oxide and the reaction temperature (°C), respectively. In Figure 1, the curve A represents the selectivity; the curve B, the reaction temperature corresponding to the curve A; and the black points C and D, the selectivity obtained under the conditions of Comparative Example 9 and the reaction temperature employed.

It is seen from the results shown by the curves A and B of Figure 1 that when the amount of sodium added exceeds 1000 ppm, especially about 1100 ppm, based on the catalyst, the catalyst of this invention gives a markedly increased selectivity for ethylene oxide at relatively low reaction temperatures. It is also seen from Figure 1 that when the amount of sodium added is within the range of 1200 ppm to 1% by weight, ethylene oxide can be produced at a high selectivity at low temperatures, and therefore amounts of sodium within this range are preferred.

### Example 11

Thirty grams of an alpha-alumina carrier (SA-5561) was dipped in 50 ml of an aqueous solution containing 2.0 g of sodium carbonate. The excess of the solution was removed by filtration, and the dipped carrier was dried at 110°C for 2 hours in a stream of nitrogen gas to prepare a sodium carbonate-impregnated carrier.

Silver nitrate (6.0 g) and 3.4 g of potassium oxalate ($K_2C_2O_4 \cdot H_2O$) were each dissolved in 100 ml of water. The resulting aqueous solutions were then mixed, and heated in a water bath at 60°C to obtain a white precipitate of silver oxalate. Potassium was removed from the precipitate by repeating centrifugation and washing with distilled water. Separately, 6.6 ml of a 1:1 mixture of ethylenediamine and water was prepared, and with ice cooling, the silver oxalate precipitate was gradually dissolved in the mixture to prepare a silver solution. The above carrier was dipped in the resulting solution. The excess of the solution was allowed to flow out, and the dipped carrier was dried at 80°C under reduced pressure in a rotary evaporator. The dried carrier was transferred to a calcination tube, and the temperature was raised in a nitrogen stream over the course of 2 hours to 300°C at which it was calcined for an additional 2 hours. The calcined product was cooled, and dipped in 50 ml of a methanol/water mixture (water content 0.3% by weight) containing 0.05 g of CsBr. The excess of the solution was removed by filtration, and the dipped product was dried at 110°C for 2 hours in a stream of nitrogen gas to prepare a supported catalyst of this invention containing 8% by weight of Ag, 0.35% by weight of Na, 126 ppm of Cs and 76 ppm of Br.

The resulting catalyst was pulverized to a size of 9 to 28 mesh, and 10 g of the pulverized catalyst was filled in a steel reaction tube having an inside diameter of 20 mm. A gaseous mixture consisting of 30% by volume ethylene, 8% by volume of oxygen, 2 ppm of vinyl chloride and the balance being methane was passed through the reaction tube at a pressure of 9 kg/cm$^2$ and an SV of 2000$^{-1}$. At a bath temperature of 213°C, an oxygen conversion of 30% and an ethylene oxide selectivity of 81% were obtained. During the 3-week operating period, no change in the performance of the catalyst was noted.

### Example 12

A supported catalyst containing 8.0% of Ag, 0.35% by weight of Na, 126 ppm of Cs and 18 ppm of F was prepared in the same way as in Example 11 except that 0.036 g of CsF was used instead of CsBr. The catalyst was tested under the same rection conditions as in Example 11. At a reaction temperature of 216°C, the results shown in Table 3 were obtained.

TABLE 3

| Example | Composition of the catalyst | | Raw materials added | Oxygen conversion (%) | Selectivity (%) |
|---|---|---|---|---|---|
| 11 | Ag——Na——Cs——Br<br>8.0   0.35   126   76<br>wt%   wt%   ppm   ppm | | $Na_2CO_3$,<br>CsBr | 30 | 81 |
| 12 | Ag——Na——Cs——F<br>8.0   0.35   126   18<br>wt%   wt%   ppm   ppm | | $Na_2CO_3$,<br>CsF | 30 | 82 |
| 13 | Ag——Na——Cs——Br<br>7.2   0.19   126   76<br>wt%   wt%   ppm   ppm | | $NaHCO_3$,<br>CsBr | 30 | 82 |
| 14 | Ag——Na——Cs——K——F<br>8.0   0.35   126   37   18<br>wt%   wt%   ppm   ppm   ppm | | $Na_2CO_3$,<br>KF,<br>$CsNO_3$ | 30 | 81 |

Example 13

A supported catalyst containing 7.2% by weight of Ag, 0.19% by weight of Na, 126 ppm of Cs and 76 ppm of Br was prepared in the same way as in Example 11 except that 2.0 g of sodium bicarbonate was used instead of sodium carbonate. The catalyst was tested under the same reaction conditions as in Example 11. At a reaction temperature of 217°C, the results shown in Table 3 were obtained.

Example 14

A supported catalyst containing 8.0% by weight of Ag, 0.35% by weight of Na, 126 ppm of Cs, 37 ppm of K and 18 ppm of F was prepared in the same way as in Example 11 except that 0.014 g of KF and 0.046 g of $CsNO_3$ were used instead of CsBr. The catalyst was tested under the same conditions as in Example 11. At a reaction temperature of 217°C, the results shown in Table 3 were obtained.

Comparative Examples 10 to 14

Five different catalysts having the compositions shown in Table 4 were prepared in the same way as in Example 11. These catalysts were each tested under the same reaction conditions as in Example 11 except that the oxygen conversion was set at 50%.

The results are shown in Table 4 together with those obtained in Example 12. It is seen that the selectivity of the catalyst of this invention is superior.

TABLE 4

| Runs | | Composition of the catalyst | | | | Raw materials added | Oxygen conversion (%) | Selectivity (%) |
|---|---|---|---|---|---|---|---|---|
| Example 12 | | Ag——Na——Cs——F<br>8.0   0.35   126   18<br>wt%   wt%   ppm   ppm | | | | Na$_2$CO$_3$,<br>CsF | 50 | 79 |
| Comparative Example | 10 | Ag——Na<br>8.0   0.35<br>wt%   wt% | | | | Na$_2$CO$_3$ | 50 | 72.8 |
| | 11 | Ag——Cs——Br<br>8.0   126   76<br>wt%   ppm   ppm | | | | CsBr | 50 | 70.3 |
| | 12 | Ag——Na——Cs——I<br>8.0   0.35   126   120<br>wt%   wt%   ppm   ppm | | | | Na$_2$CO$_3$,<br>CsI | 50 | 60 |
| | 13 | Ag——Na——Cs——Br<br>8.0   0.01   50   30<br>wt%   wt%   ppm   ppm | | | | Na$_2$CO$_3$,<br>CsBr | 50 | 74.5 |
| | 14 | Ag——Na——Cs——F<br>8.0   0.01   50   7<br>wt%   wt%   ppm   ppm | | | | Na$_2$CO$_3$,<br>CsF | 50 | 74.0 |

Example 15

A supported catalyst comprising 8.4% by weight of Ag, 2500 ppm of Na, 160 ppm of Cs, 21 ppm of Cl and 47 ppm of Br was prepared in the same way as in Example 7, and tested under the same reaction conditions as in Example 7. At a reaction temperature of 220°C, an oxygen conversion of 40% and a selectivity of 81.1% were obtained.

Example 16

A supported catalyst comprising 8.4% by weight of Ag, 2500 ppm of Na, 160 ppm of Cs, 47 ppm of Br and 11 ppm of F was prepared in the same way as in Example 7, and tested under the same reaction conditions as in Example 7. At a reaction temperature of 216°C, an oxygen conversion of 30% and a selectivity of 82.5% were obtained.

**Claims**

1. A catalyst for the production of ethylene oxide by oxidation of ethylene comprising
(A) silver,
(B) (1) sodium and (2) cesium as a cationic component, and
(C) at least one halogen element selected from the group consisting of chlorine, bromine and fluorine as an anionic component, characterized in that
(1) said sodium is contained in an amount of more than 1000 ppm (mg/kg of catalyst) based on the catalyst,
(2) said cesium is contained in an amount of from 10 ppm to 0.5% by weight based on the catalyst, and
(3) said halogen element is contained in an amount of from 5 ppm to 0.1% by weight based on the catalyst.

2. The catalyst of claim 1 wherein the amount of sodium is in the range of from 1100 ppm to 1.50% by weight based on the catalyst.

3. The catalyst of claim 1 wherein the amount of sodium is in the range of from 1200 ppm to 1% by weight based on the catalyst.

4. The catalyst of any one of claims 1 to 3 wherein silver, sodium, cesium and halogen element as

# 0 059 422

catalyst ingredients are supported on a porous refractory carrier having a surface area of 0.05 to 10 m²/ g and an apparent porosity of at least 15%.

5. A process for the production of ethylene oxide, which comprises contacting ethylene and a gas containing molecular oxygen at a temperature of 180 to 300°C and a pressure of 1 to 35 kg/cm² with a catalyst comprising

(A) silver,

(B) (1) sodium and (2) cesium as a cationic component, and

(C) at least one halogen element selected from the group consisting of chlorine, bromine and fluorine as an anionic component, characterized in that a catalyst is used in which

(1) said sodium is contained in an amount of more than 1000 ppm (mg/kg of catalyst) based on the catalyst,

(2) said cesium is contained in an amount of from 10 ppm to 0.5% by weight based on the catalyst, and

(3) said halogen element is contained in an amount of from 5 ppm to 0.1% by weight based on the catalyst.

## Patentansprüche

1. Katalysator zur Herstellung von Ethylenoxid durch Oxidation von enthaltend

(A) Silber,

(B) (1) Natrium und (2) Cäsium als eine kationische Komponente und

(C) mindestens ein Halogenelement, ausgewählt aus der Gruppe, bestehend aus Chlor, Brom und Fluor als anionische Komponente,

dadurch gekennzeichnet, daß

(1) das Natrium in einer Menge von mehr als 1000 ppm (mg/kg des Katalysators), bezogen auf den Katalysator, enthalten ist,

(2) das genannte Cäsium in einer Menge von 10 ppm bis 0,5 Gew.-%, bezogen auf den Katalysator, enthalten ist, und daß

(3) das genannte Halogenelement in einer Menge von 5 ppm bis 0,1 Gew.-% bezogen auf den Katalysator, enthalten ist.

2. Katalysator nach Anspruch 1, dadurch gekennzeichnet, daß die Menge von Natrium in Bereich von 1100 ppm bis 1,50 Gew.-%, bezogen auf den Katalysator, liegt.

3. Katalysator nach Anspruch 1, dadurch gekennzeichnet, daß die Menge von Natrium im Bereich von 1200 ppm bis 1 Gew.-%, bezogen auf den Katalysator, liegt.

4. Katalysator nach einem der Ansprüche 1 bis 3, dadurch gekennzeichnet, daß das Silber, das Natrium, das Cäsium und das Halogenelement als Katalysatorbestandteile auf einem porösen feuerfesten Träger mit einer spezifischen Oberfläche von 0,05 bis 10 m²/g und einer scheinbaren Porosität von mindestens 15% aufgebracht sind.

5. Verfahren zur Herstellung von Ethylenoxid, bei dem man Ethylen und ein molekularen Sauerstoff enthaltendes Gas bei einer Temperatur von 180 bis 300°C und einem Druck von 1 bis 35 kg/cm² mit einem Katalysator, der

(A) Silber,

(B) (1) Natrium und (2) Cäsium als seine kationische Komponente und

(C) mindestens ein Halogenelement, ausgewählt aus der Gruppe, bestehend aus Chlor, Brom und Fluor, als anionische Komponente enthält,

kontaktiert, dadurch gekennzeichnet, daß man einen Katalysator verwendet, bie dem

(1) das genannte Natrium in einer Menge von Mehr als 1000 ppm (mg/kg des Katalysators), bezogen auf den Katalysator, enthalten ist,

(2) das genannte Casium in einer Menge von 10 ppm bis 0,5 Gew.-%, bezogen auf den katalysator, enthalten ist, und

(3) das genannte Halogenelement in einer Menge von 5 ppm bis 0,1 Gew.-%, bezogen auf den Katalysator, enthalten ist.

## Revendications

1. Un catalyseur pour la production d'oxyde d'éthylène par oxydation de l'éthylène, comprenant:

(A) de l'argent,

(B) (1) du sodium et (2) du césium comme composant cationique, et

(C) comme composant anionique un ou plusieurs halogènes choisis parmi le chlore, le brome et le fluor, catalyseur caractérisé en ce que (1) la proportion du sodium est supérieure à 1000 parties en poids par million de parties du catalyseur (ppm), (2) la proportion du césium est de 10 ppm à 0,5 % du poids de catalyseur, et (2) la proportion du ou des halogènes est de 5 ppm à 0,1 % du poids du catalyseur.

2. Catalyseur selon la revendication 1 dans lequel la proportion de sodium est de 1100 ppm à 1,50 % du poids du catalyseur.

3. Catalyseur selon la revendication 1 dans lequel la proportion de sodium est de 1200 ppm à % du poids du catalyseur.

**0 059 422**

4. Catalyseur selon l'une quelconque des revendications 1 à 3, dans lequel l'argent, le sodium, le césium et l'halogène sont déposés sur un support réfractaire poreux ayant une surface spécifique de 0,05 à 10 $m^2$/g et une porosité apparente d'au moins 15 %.

5. Un procédé de production d'oxyde d'éthylène selon lequel on met en contact de l'éthylène et un gaz contenant de l'oxygène moléculaire, à une température de 180 à 300°C et sous une pression de 1 à 35 bars, avec un catalyseur comprenant:

(A) de l'argent,

(B) (1) du sodium et (2) du césium comme composant cationique, et

(C) comme composant anionique un ou plusieurs halogènes choisis parmi le chlore, le brome et le fluor, procédé caractérisé en ce que l'on emploie un catalyseur dans lequel

(1) la proportion de sodium est supérieure à 1000 parties par million de parties du catalyseur (ppm),

(2) la proportion de césium est de 10 ppm à 0,5 % du poids du catalyseur, et

(3) la proportion de l'halogène ou des halogènes est de 5 ppm à 0,1 % du poids du catalyseur.

11

# FIG. 1

0 059 422